# EUROPEAN PATENT APPLICATION

(11) **EP 4 494 556 A1**
(43) Date of publication of application: **22.01.2025**
(21) Application number: 23186908.2
(22) Date of filing: 21.07.2023
(51) Int. Cl.: A61B 5/11, A61B 5/00, G01L 1/00

(54) **METHOD AND TECHNICAL DEVICE, ESPECIALLY SMART DEVICE, IN PARTICULAR A SMART WATCH, FOR DETERMINING AT LEAST ONE MASS**

(71) Applicant: Max-Planck-Gesellschaft zur Förderung der Wissenschaften e.V., 80539 München (DE)
(72) Inventor: The designation of the inventor has not yet been filed
(74) Representative: Bals & Vogel Patentanwälte PartGmbB

(57) **Abstract**

The invention relates to a method for determining at least one mass (m) that is lifted and/or carried by a user (B), with the aid of a technical device (100), preferably in the form of a smart watch and/or a sports wristband, the technical device (100) having at least one sensor (10) that is configured to measure at least one dynamic motion parameter,
wherein sensor signals of the at least one sensor (10), are processed and/or analyzed to determine at least one mass (m) lifted and/or carried by the user (B).

## Description

The invention relates to method and a technical device, especially a smart device, preferably in the form of a smart watch and/or a sports wristband, for a user to determine at least one mass that is lifted and/or carried by the user. Furthermore, the invention relates to a corresponding use of a corresponding technical device, especially a smart device, to determine at least one mass that is lifted and/or carried by a user of the technical device, especially the smart device. Especially, the invention relates to a corresponding method for determining at least one mass, in particular a mass that is lifted and/or carried by a user, using a corresponding technical device, especially a smart device. Furthermore, the invention relates to a corresponding computer program product for carrying out a corresponding method, a corresponding storage medium on which a corresponding computer program product is stored, and a corresponding data carrier signal that transmits a corresponding computer program product. Furthermore, the invention relates to a corresponding computing unit for performing a corresponding method, wherein, in particular, the computing unit may be used in a corresponding technical device, especially a smart device.

Many working people, e.g., in moving companies, craftsmen's businesses, and/or in care professions, are confronted with lifting and carrying heavy loads by their own muscle power and without mechanical aids. In addition to work activities that involve lifting and carrying heavy loads, people also regularly move heavy loads in their private lives, often unconsciously. Lifting a weight may quickly cause significant flexion of the lumbar spine. Frequent bending and stretching of the lumbar spine under load may lead to herniated discs and back pain.

The determination of a mass, especially without a special balance, is not a trivial problem. Often, the determination of a mass eludes human intuition and, like brightness or volume, may only be estimated with difficulty and inaccuracy.

Yet this question often arises in everyday life - especially when it comes to protection against overload:
- How heavy is the hiking backpack I carry all weekend?
- How many cases of beer are good for my back and my health in the long term?
- How well may I still hold my grandchildren?

Basically, scales, such as suitcase scales or baby scales, are known. Often, such scales are not always handy, especially in domestic and everyday use.

It is therefore the task of the invention to at least partially overcome at least one of the disadvantages described above. In particular, it is the task of the invention to provide a method and a technical device, especially a smart device, preferably in the form of a smart watch and/or a sports wristband, for a user to determine at least one mass that may be lifted and/or carried by the user. Preferably, it is the task of the invention to allow a quick and easy determination of a mass, preferably without any additional equipment, such as a special scale, preferably with the help of existing resources that the user may carry with him, in particular in domestic and everyday use, such as a corresponding technical device, especially a smart device. Preferably, it is the object of the invention to provide overarching utility and enhanced functionality in a corresponding technical device, especially a smart device. Especially, it is an object of the invention to provide a universal method to determine at least one mass lifted and/or carried by a user of the technical device, especially the smart device. In addition, it is an object of the invention to provide a corresponding computer program product for performing a corresponding method, a corresponding storage medium on which a corresponding computer program product is stored, and a corresponding data carrier signal that transmits a corresponding computer program product. Furthermore, it is an object of the invention to provide a corresponding computing unit for carrying out a corresponding method, wherein, in particular, the computing unit may be used in a corresponding technical device, especially a smart device.

The foregoing task is solved by a method and a technical device, especially a smart device, preferably in the form of a smart watch and/or a sports wristband, for a user for determining at least one mass that may be lifted and/or carried by the user, having the features of the independent device claim. Furthermore, the foregoing task is solved by a corresponding use of a corresponding technical device, especially a smart device, to determine at least one mass that is lifted and/or carried by a user of the technical device. Furthermore, the foregoing task is solved by a corresponding method for determining at least one mass that may be lifted and/or carried by a user, by means of a corresponding technical device, especially a smart device, having the features of the independent method claim. Furthermore, the foregoing task is solved by a corresponding computer program product for carrying out a corresponding method, a corresponding storage medium on which a corresponding computer program product is stored, and a corresponding data carrier signal which transmits a corresponding computer program product. Furthermore, the foregoing task is solved by a corresponding computing unit for carrying out a corresponding method having the features of the subordinate device claim, wherein in particular the computing unit may be used in a corresponding technical device.

According to an aspect, the invention provides a technical device, especially a smart device, preferably in the form of a smart watch and/or a wristband, for a user to determine at least one mass that may be lifted and/or carried by the user,
comprising:
- at least one sensor, especially one motion sensor that is configured to measure at least one dynamic motion parameter, and
- a computing unit,
wherein the computing unit is specifically arranged to process (and/or analyze) sensor signals of the at least one sensor and, depending on the processing (and/or analysis), to determine the at least one mass lifted and/or carried by the user.

A technical device, especially a smart device in the context of the present disclosure may be understood to mean any smart device that a user may carry, such as a cell phone, smart watch, wristband, sports wristband, heart rate monitor, ankle bracelet, smart garment, ring, gloves, camera device, smart glasses, head phones etc., having some logic and sensoria.

A sensor, especially a motion sensor, within the scope of the present disclosure may be understood as any sensor that may measure a dynamic motion parameter, such as a position, a velocity and/or an acceleration. A sensor within the scope of the present disclosure may be understood as any sensor capable of measuring a position, a motion, an acceleration, an inclination, a force, a rotation, an angular momentum, a field parameter (for example magnetic field), a gravitation parameter, or the like. The sensor within the scope of the present disclosure may be an electromechanical sensor and/or an electromagnetic sensor. The sensor within the scope of the present disclosure may sense a dynamic motion parameter in at least 2 or 3 directions, or may be configured as a 2D or 3D sensor.

A mass in the context of the present disclosure may be understood as an object having a certain weight. A mass in the context of the present disclosure may be understood as a load or a weight.

With the help of the proposed technical device, a quick and easy determination of a mass may be made possible. No additional equipment is required for this - a smartwatch may be used, for example.

The idea is thus to capture and analyze a dynamic and/or a type of a movement when a mass is moved (e.g., lifted or "weighed" in the hand, i.e., moved up and down). Such a characteristic motion may be modeled and learned by an artificial intelligence. Such a characteristic motion may be modeled for at least one mass or for different masses. Such a characteristic motion may be analyzed user-specifically, for example by calibrating and/or normalizing sensor signals. A calibration may be done user specific. For this purpose, for example, a training run (which may also be called calibration run) may be carried out with the aid of the technical device.

A significant advantage of the invention is the universal availability of such a technical device, since the technical device may always be at hand or always available, for example, when the smartwatch is worn on the wrist.

Moreover, with the help of such a technical device, an overarching benefit and enhanced functionality may be provided. Advantageously, such a technical device may be further developed as an assistant for analyzing personal loads when lifting and carrying heavy loads.

In addition, such a technical device may record unconscious lifting and carrying activities over the course of the day, add them up, and send a warning message if the loads are too high, for example, with the aid of the same technical device or with the aid of another of the user's mobile devices. In this way, the user may raise his or her own awareness of lifting and carrying loads in everyday life and, if necessary, use his or her actions to prevent illnesses such as slipped discs and back pain.

In addition, such a technical device may be used to create stress profiles and transmit them to a health care provider, such as an employer and/or a physician.

Furthermore, with the help of such a technical device, an advantageous interaction with the user may be enabled. The user may be informed about a determined mass and/or a corresponding load profile. The user may be consulted to calibrate sensor signals and/or to record different signal examples and/or frequency examples, e.g., for one mass or several different masses. For this purpose, the technical device may be designed with a user interface.

Furthermore, it is conceivable that such a technical device may be individualized for a particular user, for example as a personal smartwatch. Furthermore, it is conceivable that such a technical device may be used as a universal measuring instrument, for example in a fitness studio or in a doctor's office.

Furthermore, it may be provided that the at least one sensor comprises at least one acceleration sensor, at least one force sensor, at least one inclination sensor, at least one magnet field sensor, at least one gravitational sensor and/or at least one combined sensor. With the aid of such sensors, it is possible to detect a dynamic of a movement when a mass is moved. Laws of motion may simply be used to analyze the motion, so that the analysis may be performed with little computational effort. One possibility may be to analyze the motion of a mass under the influence of a measured force, such as a spring force. The acceleration of an object is directly proportional to the force acting on it and inversely proportional to its mass. If the sensor may detect a force acting on an object and/or the resulting acceleration, the mass may be determined as force divided by acceleration. Another possibility may be to analyze the motion of a mass using a measured velocity. If the velocity of an object is known, its momentum may also be determined, which is directly proportional to both mass and velocity. If the sensor may measure a velocity and/or the resulting momentum, the mass may be determined as momentum divided by velocity.

Furthermore, it may be provided that the at least one sensor may have at least one electromechanical sensor, in particular a piezoelectric sensor and/or a capacitive sensor, at least one electromagnetic sensor, in particular a rotational acceleration sensor and/or an inductive sensor. With the aid of such sensors, simple and inexpensive detection of a movement may be made possible.

Furthermore, it may be provided that the at least one sensor may comprise at least one 3D sensor. In this way, an improved analysis of a dynamic of a movement may be enabled, which may be analyzed in different directions.

Furthermore, a storage unit may be provided, wherein the storage unit has a permanent storage area. Preferably, the permanent storage area may be used to store a code that may be used to perform a corresponding method for determining a mass. Further, the permanent storage area may be used to store a map and/or an artificial neural network that may enable a simple and effective analysis of a dynamic and/or a type of a detected motion.

Furthermore, a storage unit may be provided, wherein the storage unit may have a volatile storage area. Preferably, the permanent storage area may serve as a working memory for the computing unit.

Furthermore, a storage unit may be provided, wherein the storage unit has at least one first, in particular permanent, storage area in which a map is stored which has different signal examples and/or frequency examples of, in particular calibrated and/or normalized, sensor signals of the at least one sensor for corresponding masses. Furthermore, it is conceivable that different signal examples and/or frequency examples of, in particular calibrated and/or normalized, sensor signals of the at least one sensor for corresponding masses may be stored for at least one user or for several different users. Furthermore, it is conceivable that different signal examples and/or frequency examples of, in particular calibrated and/or normalized, sensor signals of the at least one sensor may be stored for different types of motion, e.g., for one type of motion - lifting and/or for another type of motion - carrying while walking, etc. With the aid of a map, a fast analysis of a dynamic and/or a type of movement may be made possible with little computing effort. In doing so, the computing unit may search for patterns in the new sensor signals that are comparable with stored signal examples or frequency examples in order to determine a corresponding mass.

Furthermore, a storage unit may be provided, wherein the storage unit has at least one second, in particular permanent, storage area in which a, preferably self-learning, artificial neural network is stored, which has been specially trained and/or may be trained to determine a corresponding mass on the basis of, in particular calibrated and/or normalized, sensor signals of the at least one sensor. In this way, a particularly fast and precise analysis of a dynamic (e.g. how fast) and/or a type (e.g. lifting vs. carrying) of a movement may be made possible. Artificial neural networks may be used in an advantageous way in signal processing, in particular to perform an analysis of signal courses and/or signal frequencies. Artificial neural networks may be trained to recognize patterns in sensor signals, including frequency patterns. For example, artificial neural networks may be trained on a data set of signal data with known signal examples and/or frequency examples and then used to identify and classify similar signal examples and/or frequency examples in new signals. Advantageously, artificial neural networks may implement Fourier transforms and wavelet transforms that may be used in an advantageous way to analyze the frequency content of signals. These techniques are widely used in signal processing to extract information from signals in different domains, such as the time domain, the frequency domain, and the time-frequency domain.

Furthermore, it may be provided that the computing unit has an electronics unit that is specially set up to process sensor signals, in particular calibrated and/or normalized sensor signals, of the at least one sensor using a frequency analysis method. In this way, sensor signals may be analyzed easily and quickly using established signal processing methods.

Advantageously, the computing unit may be specially set up to analyze the frequency, the amplitude and/or the shape of, in particular calibrated and/or normalized, sensor signals of the at least one sensor. A dynamic and/or a type of a movement may be described with the help of the frequency, the amplitude and/or the shape of, in particular calibrated and/or normalized, sensor signals of the sensor. A dynamic and/or a type of a movement may in turn be specific to the mass of a moving object. Depending on the frequency, the amplitude and/or the shape, in particular the transients and/or slope, of, in particular calibrated and/or normalized, sensor signals of the at least one sensor, a moving mass may be determined easily and quickly. For one thing, it is conceivable, for example, that if the user is walking with the mass, a relatively heavy mass may exhibit higher amplitudes in the sensor signal than a comparatively lighter mass. Further, it is conceivable that when the user is walking with the mass, a relatively heavy mass may show smaller frequencies in the sensor signal than a comparatively lighter mass. When the user lifts a mass, special ratios in the sensor signal may also occur. A rather flat transient in the sensor signal may indicate that a relatively heavy mass is being lifted. A rather steep transient in the sensor signal may be a sign that a relatively light mass is being lifted, etc.

In addition, it may be provided that the computing unit has at least one filter, in particular a high-pass filter and/or low-pass filter, to process sensor signals, in particular calibrated and/or normalized sensor signals, of the at least one sensor for determining whether the user is carrying and/or lifting a mass. With the help of filters, threshold values for different signal frequencies may be set easily and quickly. In this way, frequencies that are too high and/or too low in the sensor signals may be detected easily and quickly.

On the one hand, it may be advantageous if the computing unit may be specially set up to process (or analyze) different signal examples and/or frequency examples of, in particular calibrated and/or normalized, sensor signals of the at least one sensor with the aid of a characteristic map in order to determine the mass.

On the other hand, it may be advantageous if the computing unit may be specially set up to process (or analyze) sensor signals, in particular calibrated and/or normalized sensor signals, from the sensor using an artificial neural network in order to determine the mass.

Furthermore, it may be provided that the computing unit is specifically set up to calibrate and/or normalize sensor signals of the at least one sensor as a function of at least one vital parameter of the user, in particular comprising: a weight, a body height, a body density, a BMI index, a pulse, a heartbeat, a blood volume, a blood pressure, an oxygen saturation in the blood and/or a temperature. In this way, user-specific analysis of sensor signals may be enabled. Differently built and/or trained users may lift and/or carry comparable masses in a different manner. For example, a well-trained furniture mover may carry heavy loads more easily than a user who does not often lift and/or carry heavy loads due to his job, e.g., office work. But also a momentary condition of one and the same user, e.g., after a recovery break or after a long working day, may influence the sensor signals. Such user-specific factors may be mapped in a beneficial way with the help of vital parameters and taken into account in the signal analysis in order to determine the masses equally correctly for different users and/or for the same user in different situations. Different vital parameters of the user can, for example, be provided as inputs to a corresponding neural network. But also a consideration of vital parameters of the user in the context of a map is also conceivable.

Furthermore, it may be provided that the computing unit is specifically set up to process and/or analyze sensor signals of the at least one sensor depending on at least one geographical position of the user. In this way, the geographic position of the user may be taken into account in the signal analysis and thus in the determination of a mass. If the geographic position of the user changes, it is more likely that a movement type - wearing while walking may be inferred. If the geographic position of the user does not change, it is more likely to infer a movement type - lifting masses. This may simplify an assignment of relevant patterns in the sensor signal to existing signal examples or frequency examples.

Advantageously, the computing unit may be specifically arranged to control at least one sensor of the technical device to measure and/or determine at least one vital parameter and/or at least one geographical position of the user. In this way, the resources of the technical device may be utilized in an advantageous manner to enable advanced function at the technical device.

There are several sensors that may be used to measure a user's vital signs and that may be controlled by the computing unit for measuring vital signs. A heart rate sensor: this sensor may measure the user's heart rate by detecting, for example, pulsations in the blood vessels. An electrocardiogram sensor (or ECG sensor): This sensor may measure the electrical activity of the heart. A blood pressure sensor: This sensor may measure the pressure of the blood in the arteries. A pulse oximeter: This sensor may measure the oxygen saturation in the blood. A temperature sensor: This sensor may measure the user's body temperature.

In wearable smartwatches, the following sensors may be used to measure the user's vital signs. A heart rate sensor: This sensor may measure the user's heart rate. An accelerometer: this sensor may be used to track the user's movements and estimate their activity level. A gyroscope: This sensor may be used to track the user's orientation and movements. A GPS sensor: This sensor may be used to determine the user's location and calculate the distance traveled. A barometer: This sensor may be used to measure the user's altitude, which may be used to estimate activity levels. Smartwatches in accordance with the present disclosure may further have sensors such as ECG, blood pressure, and temperature sensors, which may be used to more closely monitor the user's vital signs.

Furthermore, it may be provided that the computing unit is specifically set up to initiate and/or perform at least one training run in order to obtain at least one calibration signal of the at least one sensor for a motion of the user without a mass, which may preferably be used to calibrate and/or normalize sensor signals of the at least one sensor for a motion of the user with a mass. In order to initiate a training run, it may advantageously be provided that the user is made aware by the computing unit that a training run is to be actively started by him. To start the training run by the user, for example, a manual input, a keystroke or the like may be provided. To initiate a training run, it may further be provided that the computing unit may start the training run automatically, e.g., when a certain event has taken place, e.g., when the technical device is activated, when the function for determining the mass is activated and/or when a new user has logged on to the technical device. In order to perform a training run, the computing unit may automatically trigger the training run by, for example, controlling the sensor accordingly and/or interactively guiding the user through the training run to support the training run.

Furthermore, it may be provided that the computing unit is specifically set up to initiate and/or perform at least one or more measurements in order to obtain different signal examples or frequency examples of, in particular, calibrated and/or normalized, sensor signals of the at least one sensor for corresponding masses, preferably for at least one user or for several different users. In this way, on the one hand, training data, for example for an artificial neural network, may be collected. On the other hand, data for a characteristic map may be collected in this way. Different signal examples or frequency examples of, in particular calibrated and/or normalized, sensor signals of the at least one sensor for corresponding masses may advantageously help to find known patterns in the new sensor signals and to assign a corresponding mass to the found patterns.

Advantageously, the technical device may further comprise at least one user-side interface. The at least one user-side interface may, for example, comprise a display. However, the at least one user-side interface may also comprise buttons, keys, touch-sensitive surfaces, etc.

According to a further advantage, the computing unit may be specifically arranged to control the at least one user-side interface in such a way that, with the aid of the user, at least one training run may be started and/or performed to calibrate and/or normalize sensor signals of the at least one sensor for a movement of the user with a mass, preferably for at least one user or for several different users. In this way, the user may be involved to set up the proposed functionality. Advantageously, the computing unit may thereby enable interaction with the user via the at least one user-side interface.

Furthermore, it may be advantageous that the computing unit is specifically set up to control the at least one user-side interface in such a way that, with the help of the user, at least one or more measurements may be started and/or performed to obtain different signal examples or frequency examples of, in particular, calibrated and/or normalized, sensor signals of the at least one sensor for corresponding masses, preferably for at least one user or for several different users. Also in this way, the user may be involved to set up the proposed functionality. Advantageously, the computing unit may thereby enable interaction with the user via the at least one user-side interface.

A valuable advantage may further result from the fact that the computing unit is specifically set up to create and/or analyze load profiles, preferably for at least one user or for several different users. In these ways, the utility and functionality of the technical device may be significantly increased.

Furthermore, it is conceivable that the load profiles are created regularly, for example, when a mass is lifted and/or carried, periodically, for example every day, and/or event-controlled, for example by activation, for example by the user. In this way, the utility and functionality of the technical device may be made flexible.

In order to communicate with the user at least one user-side interface, for example comprising a display, preferably a touch display and/or at least one microphone, may be provided. Preferably the at least one user-side interface may be specially arranged to capture a user input, especially a voice command and/or a touch command, which may be used to start mass determining. For this aim, the at least one user-side interface may control the computing unit after corresponding user input to determine at least one mass lifted and/or carried by the user and/or at least one load profile of the user.

In order to increase the usability of the technical device, the at least one user-side interface may be at least partially implemented in an external device, for example a mobile device of a user, such as a smart phone, smart glasses, a head up display, a virtual reality device, an augmented reality device and/or a mixed technology device.

In order to visualize the functionality of the technical device, the computing unit may be specifically arranged to control the at least one user-side interface to output at least one particular mass lifted and/or carried by the user and/or at least one load profile of the user. In this way, the functionality of the technical device may be made understandable.

To further improve the functionality of the technical device, the computing unit may be specifically configured to control the at least one user-side interface to output an acoustic, optical and/or haptic feedback to the user when at least one mass and/or when at least one load profile exceeds a certain risk threshold for the user. Advantageously, the feedback may comprise at least one or more warning levels. Advantageously, a risk threshold may be determined as a function of at least one vital parameter of the user. For this purpose, the computing unit may perform a special calculation. With the aid of the feedback provided to the user, the user may be warned and protected against damage to health.

To further enhance the functionality of the technical device, the computing unit may be specifically adapted to communicate at least one determined mass lifted and/or carried by the user and/or at least one load profile of the user to a mobile device of the user and/or to a health service, for example, at an employer and/or a doctor. In this way, the technical device may become a part of a system that protects and even promotes the user's health.

To further enhance the functionality of the technical device, the computing unit may be specifically configured to send out an emergency call when at least one mass and/or at least one stress profile exceeds a certain risk threshold for the user. The distress call may be sent out to a caregiver, a doctor, a hospital, and/or an emergency response service or the like. In this way, it may be ensured that the user is helped in an emergency.

According to another aspect of the invention, it is generally proposed to: use of a technical device, especially a smart device, which may be configured, for example, as described above, to determine at least one mass lifted and/or carried by a user of the technical device. In this regard, the invention recognizes that such technical devices are most often worn close to the user's body, for example, on the wrist, chest, head, or other body location, and have sensors that may detect the dynamics and/or nature of a movement of the user. The invention also recognizes that motion detection using physical laws may enable determination of lifted and/or carried masses or weights. The invention takes advantage of these findings and proposes the use of a technical device for mass determination. The technical device may be used in daily use, for example, for measuring a weight of a suitcase, a crate, a moving box, or the like. The technical device may be used for kitchen aids, for example to weigh some foods, etc. The technical device may be used for different purposes, for example to control overloading of a transport device, vehicle, etc. The technical device may further be used to estimate a weight of a child being lifted and/or carried on the arm. The technical device may be used as a fitness device for measuring masses, for example in a gym when lifting weights. The technical device may be used as a health monitoring device for measuring mass, for example in a work environment involving lifting and/or carrying weights.

According to another aspect of the invention, it is generally proposed to: use of a technical device, especially a smart device, which may be configured, for example, as described above, for measurement of a liquid intake.

According to an aspect, the invention provides a method for determining at least one mass that may be lifted and/or carried by a user, by means of a technical device, preferably in the form of a smart watch and/or a sports wristband, for the user, the technical device comprising at least one sensor that is configured to measure at least one dynamic motion parameter, wherein sensor signals of the at least one sensor are processed and/or analyzed to determine at least one mass lifted and/or carried by the user. By means of the method, the same advantages described above in connection with the technical device may be achieved. These advantages are fully referred to herein.

Advantageously, sensor signals, in particular calibrated and/or normalized sensor signals, of the at least one sensor may be processed with the aid of a frequency analysis method to determine at least one mass. In this way, established methods from frequency analysis may be used to process and/or analyze the sensor signals of the at least one sensor.

To determine at least one mass, the frequency, the amplitude, and/or the shape of, in particular calibrated and/or normalized, sensor signals of the at least one sensor can/may be analyzed in order to determine the mass in particular as a function of the frequency, the amplitude, and/or the shape, in particular of the transients and/or slopes, of, in particular calibrated and/or normalized, sensor signals of the at least one sensor. In this way, patterns in the sensor signals may be detected that may indicate a determined mass.

For simplicity, at least one filter, for example, a high-pass filter and/or a low-pass filter, may be used to determine whether the user is carrying and/or lifting a mass. If lifting and/or carrying a mass is associated with certain frequencies that are too high and/or too low, such frequencies may be safely and reliably detected by simple electrotechnical circuits in the form of filters.

On the one hand, it is conceivable that different signal examples or frequency examples of, in particular calibrated and/or normalized, sensor signals of the at least one sensor are processed with the aid of a map in order to determine the mass. In this way, a simple and fast assignment of corresponding masses to the similar signal examples or frequency examples may be made possible.

On the other hand, it is conceivable that sensor signals, in particular calibrated and/or normalized sensor signals, of the at least one sensor are processed with the aid of an artificial neural network in order to determine the mass. In this way, a simple and fast analysis of sensor signals may be made possible.

Advantageously, sensor signals of the at least one sensor may be calibrated and/or normalized depending on at least one vital parameter of the user, in particular comprising: a weight, a height, a body density, a BMI index, a pulse, a heartbeat, a blood volume, a blood pressure, an oxygen saturation in the blood and/or a temperature. In this way, the mass determination may be user-specific.

Furthermore, sensor signals of the at least one sensor may be processed and/or analyzed depending on at least one geographical position of the user. In this way, in particular, the type of movement of the user may be verified. For example, this may identify whether the user is lifting a mass and/or carrying a mass while walking. In this way, the processing and/or analysis of sensor signals may be improved.

Advantageously, at least one sensor of the technical device may be used to measure and/or determine at least one vital parameter and/or at least one geographical position of the user. In this way, the existing resources of the technical device may be used in an improved manner.

In order to refine the mass determination, it may be provided that at least one training pass is performed to obtain at least one calibration signal from the at least one sensor for a motion of the user without a mass. Preferably, the calibration signal may be used to calibrate and/or normalize sensor signals of the at least one sensor for a motion of the user with a mass. Thus, the determination of the mass may be precise and/or user-specific.

In order to enable accurate mass determination, it may be provided that at least one or more measurements are performed to obtain different signal examples or frequency examples of, in particular calibrated and/or normalized, sensor signals of the at least one sensor for corresponding masses, preferably for at least one user or for several different users. In this way, training data and/or map data may be obtained for signal analysis.

The method may be used to create and/or analyze load profiles. Load profiles may preferably be created for at least one user or for several different users. In terms of control technology, it is conceivable that load profiles are created regularly, e.g., when a mass (m) is lifted and/or carried, periodically, e.g., every day, and/or event-controlled, e.g., by activation, e.g., by the user.

The method may be used to create and/or analyze motion profiles for measurement of a liquid intake. Motion profiles may preferably be created for at least one user or for several different users. In terms of control technology, it is conceivable that load profiles are created regularly, e.g., when a mass is lifted and/or carried, periodically, e.g., every day, and/or event-controlled, e.g., by activation, e.g., by the user.

In order to make the functionality recognizable to the user, it may be provided that at least one determined mass lifted and/or carried by the user and/or at least one load profile of the user and/or a measured liquid intake are output to the user.

In order to start determining at least one mass lifted and/or carried by the user a user input, especially a voice command and/or a touch command, may be captured. Further, a user input, especially a voice command and/or a touch command, may be captured to start creating and/or analyzing at least one load profile of the user and/or to start a measurement of a liquid intake. Thus, the user may actively use the technical device for the aims of mass determining and/or creating and/or analyzing lode profiles.

For reasons of safety and/or health promotion, it may be provided that an acoustic, optical and/or haptic feedback is output to the user if at least one determined mass and/or if at least one created load profile exceeds a certain risk threshold for the user. Advantageously, the feedback may comprise at least one or more warning levels.

To increase the usefulness of the method, it may be provided that at least one determined mass lifted and/or carried by the user and/or at least one load profile of the user are/is transmitted to a mobile device of the user and/or to a health service, e.g., at an employer and/or a physician.

For safety reasons, it may be provided that an emergency call is sent out if at least one determined mass and/or if at least one created load profile exceeds a certain risk threshold for the user.

According to another aspect, the invention provides a computer program product comprising instructions that, when the computer program is executed by a computer, cause the computer program to perform a method that may be as described above. By means of the computer program product, the same advantages described above in connection with the technical device and/or with the method may be achieved. These advantages are fully referred to herein.

According to another aspect, the invention provides a storage medium on which a corresponding computer program product is stored. By means of the storage medium, the same advantages described above in connection with the technical device and/or with the method may be achieved. These advantages are fully referred to herein.

According to another aspect, the invention provides a data carrier signal that transmits a corresponding computer program product. By means of the data carrier signal, the same advantages described above in connection with the technical device and/or with the method may be achieved. These advantages are fully referred to herein.

According to a further aspect, the invention provides a computing unit for determining at least one mass that may be lifted and/or carried by a user, comprising a memory device (may be understand as a storage unit as described above) and a computing device (may be understand as a computing unit as described above), wherein a code is stored in the memory device, and wherein, when the code is executed by the computing device, a method is carried out which may be executed as described above. By means of the computing device, the same advantages described above in connection with the technical device and/or with the method may be achieved. These advantages are fully referred to herein.

Advantageously, the computing unit may be specifically adapted to be used in a technical device, which may be embodied as described above, to process sensor signals from the sensor and to determine the at least one mass lifted and/or carried by the user as a function of the processing.

In order to increase computing power and capacity by performing the method, the memory device and the computing device may be at least partially implemented on an external device, like a cloud computer, computing center etc.

Further advantages, features, and details of the invention will be apparent from the following description, in which several embodiments of the invention are described in detail with reference to the drawing. The figures show:
- Fig. 1a: schematic diagram of a technical device,
- Fig. 2a: schematic representation of sensor signals and corresponding signal examples or frequency examples.

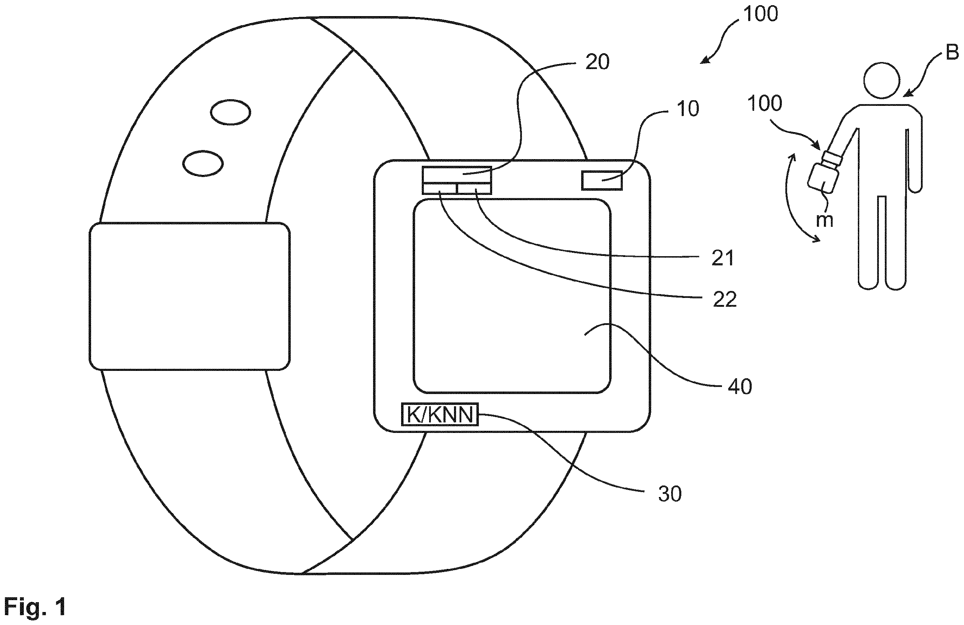

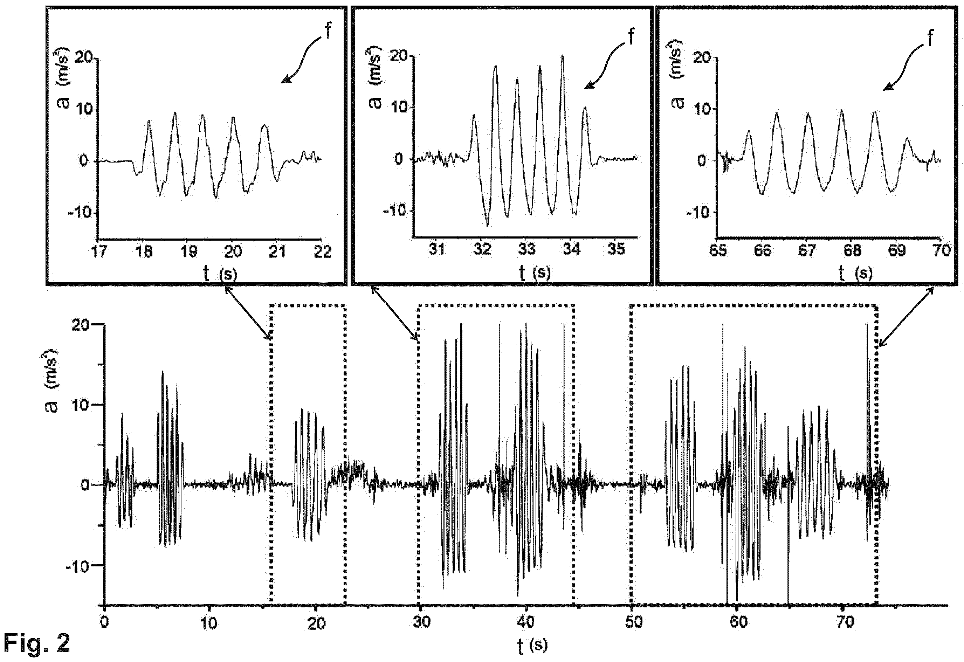

Figs. 1 and 2 serve to explain the idea of the invention.

According to an aspect, a technical device 100 is proposed, especially a smart device, preferably in the form of a smart watch and/or a wristband that a user B may wear close to the body, preferably on the wrist. The smart device 100 is specifically designed to determine at least one mass m that may be lifted and/or carried by the user B.

The technical device 100 has the following components:
- at least one sensor, especially one motion sensor 10 that is configured to measure at least one dynamic motion parameter, and
- a computing unit 30,
wherein the computing unit 30 is specifically arranged to process and/or analyze sensor signals of the at least one sensor 10 and to determine the at least one mass m lifted and/or carried by the user B as a function of the processing and/or the analysis.

The technical device 100 may in principle be embodied as a smart device of various types that a user B may carry, such as a cell phone, a smart watch, a (sports) wristband, a (sports) headband, a (sports) ankle band, a (sports) chest band, a ring, a heart rate monitor, a smart garment, etc.

The sensor 10, especially the motion sensor, in the context of the present disclosure may comprise any sensor capable of sensing a dynamic motion parameter of a user B, such as a position, a velocity and/or an acceleration, etc. The sensor 10 may comprise any sensor capable of measuring a position, a motion, an acceleration, a tilt, a force, a rotation, or the like. The sensor 10 may be implemented as an electromechanical sensor and/or an electromagnetic sensor and/or electro optical sensor. The sensor 10 may sense at least one dynamic motion parameter in at least 2 or 3 directions, or may be implemented as a 2D or 3D sensor.

A mass m in the context of the present disclosure may be understood as an object having a certain weight. However, a mass m may also be used as a synonym for a load and/or weight.

The technical device 100 is used for quick and easy determination of a mass m, without separate scales.

The idea here is to capture and analyze a dynamic and/or a type of a motion when a mass m is moved, e.g., lifted or "weighed" in the hand, i.e., moved up and down. Such a characteristic motion may be modeled and learned by an artificial neural network KNN. Such a characteristic motion may be modeled for at least one mass m or for different masses m. Such a characteristic motion may be analyzed user-specifically, for example by calibrating and/or normalizing the sensor signals. Calibrations may be done user specific. For this purpose, for example, a training run may be used, which may be performed, for example, by the technical device 100.

The technical device 100 may be used universally. Advantageously, the technical device 100 is always at hand or always available, for example, when the smartwatch is worn on the wrist.

With the aid of the technical device 100, an overarching utility and enhanced functionality may be provided. Advantageously, the smart device 100 may be further developed as an assistant for analyzing personal load when lifting and carrying loads.

The technical device 100 may record conscious and unconscious lifting and carrying activities, for example over the course of a day, add them up and, advantageously, send a warning message if the loads are too high. In this way, the user may raise his or her own awareness of lifting and carrying loads in everyday life and, if necessary, use his or her actions to prevent illnesses such as slipped discs and back problems.

The technical device 100 may also create and transmit workload profiles to a health care provider, such as an employer and/or a physician.

With the aid of the technical device 100, interaction with the user B may be enabled. The user B may be informed about a determined mass m and/or an applied load profile. The user B may be used to calibrate sensor signals and/or to create different signal examples and/or frequency examples, in particular for at least one mass or advantageously for several different masses.

The technical device 100 may be implemented with a user-side interface 40, which may be controlled accordingly by the computing unit 30.

The technical device 100 may be designed for a personal use for a particular user. The technical device 100 may further be used as a universal measurement tool, for example, in a gym or in a doctor's office.

Furthermore, the technical device 100 may comprise a storage unit 20.

In one aspect, the storage unit 20 may include a permanent storage area. The permanent storage area may be used, for example, to store a code that may be used to perform a corresponding procedure for determining a mass m. Further, the permanent storage area may be used to store a map K and/or an artificial neural network KNN that may enable a simple and effective analysis of a dynamic and/or a type of a detected motion.

Secondly, the storage unit 20 may have a volatile storage area. The permanent storage area may, for example, serve as a working memory for the computing unit 30.

The storage unit 20 may have at least one first, in particular permanent, storage area 21 in which a map K may be stored. The map K may map different signal examples and/or frequency examples f (cf. Fig. 2) of, in particular, calibrated and/or normalized, sensor signals of the at least one sensor 10 for corresponding masses m.

Different signal examples and/or frequency examples f of, in particular calibrated and/or normalized, sensor signals of the at least one sensor 10 for corresponding masses m may be stored personalized for at least one user B or universally for several different users B.

Different signal examples and/or frequency examples f of, in particular calibrated and/or normalized, sensor signals of the at least one sensor 10 for corresponding masses m may be stored for different types of motion. This means that during processing and/or analysis of sensor signals, a distinction may be made between different types of motion, e.g. between one type of motion - lifting and/or another type of motion - carrying while walking, etc.

Furthermore, the storage unit 20 may have at least one second, in particular permanent, storage area 22, in which a, preferably self-learning, preferably trained or trainable, artificial neural network KNN may be stored. The artificial neural network KNN may have been trained or be trainable specifically to be able to infer a corresponding mass m on the basis of, in particular calibrated and/or normalized, sensor signals of the at least one sensor 10. The artificial neural network KNN may perform an analysis of signal progressions and/or signal frequencies. The artificial neural network KNN may be trained to recognize patterns in the sensor signals of the at least one sensor 10, including amplitude patterns, frequency patterns signal shapes, or the like. For example, the artificial neural network KNN may be trained on a data set of signal examples and/or frequency examples and then used to identify similar signal examples and/or frequency examples in new sensor signals and assign them to a particular mass. Advantageously, the artificial neural network KNN may implement Fourier transforms and wavelet transforms that may be used in an advantageous manner to analyze the frequency content of sensor signals. Advantageously, the artificial neural network KNN may implement signal processing methods to extract information from sensor signals, e.g., amplitudes, frequencies, waveforms, etc.

The computing unit 30 may preferably have an electronics unit that is specially set up to process sensor signals, in particular calibrated and/or normalized sensor signals, of the at least one sensor 10 with the aid of at least one method for frequency analysis. The electronics unit may have electrotechnical and/or electronic components that may be used for analog signal conditioning, digital signal processing and/or evaluation.

The computing unit 30 may preferably be specifically set up to analyze the frequency, the amplitude and/or the shape of, in particular calibrated and/or normalized, sensor signals of the at least one sensor 10. A dynamic and/or a type of a movement may be described with the aid of the frequency, amplitude and/or the shape of, in particular calibrated and/or normalized, sensor signals of the sensor 10. A dynamic and/or a type of a movement may in turn be specific to the mass m of a moving object that the user B lifts or carries while walking. For example, if the user is walking with mass m, a relatively heavier mass m may cause higher amplitudes in the sensor signal than a lighter mass m in comparison. For example, if the user is running with mass m, a relatively heavy mass m may show smaller frequencies in the sensor signal than a lighter mass m in comparison. For example, if the user B is lifting a mass m, a rather flat transient in the sensor signal may indicate that a relatively heavy mass is being lifted. A rather steep transient in the sensor signal may indicate that a relatively light mass m is being lifted.

For convenience, the computing unit 30 may include at least one electrotechnical filter, for example, a high-pass filter and/or low-pass filter, to detect signals having specific frequencies and thus determine whether the user B is carrying and/or lifting a mass m. With the help of filters, threshold values for different signal frequencies may be set easily and quickly.

On the one hand, the computing unit 30 may be specifically set up to process and/or analyze different signal examples and/or frequency examples f of, in particular, calibrated and/or normalized, sensor signals of the at least one sensor 10 with the aid of a characteristic map K in order to determine the mass m.

Instead, or in addition thereto, the computing unit 30 may be specifically configured to process and/or analyze sensor signals, in particular calibrated and/or normalized sensor signals, of the at least one sensor 10 using an artificial neural network KNN in order to determine the mass m.

Advantageously, the computing unit 30 may be specifically configured to process or analyze sensor signals from the sensor 10 as a function of at least one vital parameter of the user B. The at least one vital parameter may comprise, for example: a weight, a height, a body density, a BMI index, a pulse, a heartbeat, a blood volume, a blood pressure, a blood oxygen saturation, and/or a temperature, etc. In this way, a user-specific analysis of sensor signals may be made possible, which may be individually adapted to a concrete user B. Differently built and/or trained users B may lift and/or carry comparable masses m in a different way. For example, a well trained furniture mover may carry heavy loads more easily than a clerk who does not often lift and/or carry heavy loads due to his job, e.g., office work. But also a momentary condition of one and the same user, e.g., after a recovery break or after a long working day, may influence the sensor signals. Such user-specific factors may be mapped in a beneficial way with the help of vital parameters and may be taken into account when determining the mass. Different vital parameters of the user B can, for example, be provided as additional inputs to an appropriately trained neural network and/or as additional data to a map.

The computing unit 30 may take into account at least one geographical position of the user B when processing and/or analyzing the sensor signals of the at least one sensor 10. If the geographical position of the user B changes over time, it may be more likely to infer a movement type - carrying while walking. If the geographical position of the user does not change, it is more likely to infer a movement type - lifting masses.

Preferably, the computing unit 30 may be specifically configured to appropriately control at least one sensor of the technical device 100 to measure at least one vital sign and/or at least one geographic location of the user B.

The computing unit 30 may further be specifically configured to initiate and/or perform at least one training run To initiate a training run, the computing unit 30 may, e.g., using the user-side interface 40, indicate to the user B that a training run is to be enabled or started, e.g., by a manual input, a keystroke, or the like. The computing unit may further start the training run automatically, for example, when a certain event has occurred, such as when the technical device 100 is activated, when the function for determining the mass m is activated, and/or when a new user B has logged on to the technical device 100. In order to perform a training run, the computing unit 30 may automatically control the course of the training run, for example by controlling the sensor 10 accordingly and/or by interactively guiding the user B through the training run.

The computing unit 30 may further be specifically arranged to initiate and/or perform at least one or more measurements to obtain different signal examples or frequency examples f of, in particular, calibrated and/or normalized, sensor signals of the at least one sensor 10 for corresponding masses m. The signal examples or frequency examples f may preferably be performed for at least one user B or for several different users B.

As noted above, the technical device 100 may further comprise at least one user-facing interface 40. As indicated by Fig. 1, the at least one user-facing interface 40 may comprise, for example, a display. The at least one user-facing interface 40 may further comprise buttons, keys, touch-sensitive surfaces, or the like.

The computing unit 30 may be specifically configured to control the at least one user-side interface 40 such that at least one training run may be started and/or performed with the aid of the user B.

The computing unit 30 may be specifically configured to control the at least one user-side interface 40 such that at least one or more measurements may be started and/or performed with the aid of the user B.

According to a further advantage, the computing unit 30 may be specifically set up to create and/or analyze load profiles, preferably for at least one user B or for several different users B. The load profiles may be created regularly, for example, when a mass m is lifted and/or carried, periodically, for example every day, and/or event-driven, for example by activation, for example by the user B.

To visualize the functionality of the technical device 100, the computing unit 30 may be specifically configured to control the at least one user-facing interface 40 to output at least one particular mass m lifted and/or carried by the user B and/or at least one load profile of the user B.

The at least one user-facing interface 40 may be preferably arranged to start determining at least one particular mass m lifted and/or carried by the user B and/or to create and/or to analyze at least one load profile of the user B.

To further enhance the functionality of the technical device 100, the computing unit 30 may be specifically configured to control the at least one user-facing interface 40 to output an acoustic, visual, and/or haptic feedback to the user B when at least one mass m and/or when at least one load profile exceeds a certain risk threshold for the user B. Advantageously, the feedback may comprise at least one or more warning levels. Advantageously, a risk threshold may be determined as a function of at least one vital parameter of the user, preferably by the computing unit 30.

To further enhance the functionality of the technical device 100, the computing unit 30 may be specifically configured to communicate at least one particular mass m lifted and/or carried by the user B and/or at least one load profile of the user B to a mobile device of the user B and/or to a health care provider, such as an employer and/or a physician.

To further enhance the functionality of the technical device 100, the computing unit 30 may be specifically configured to send an emergency call when at least one mass m and/or when at least one load profile exceeds a certain risk threshold for the user B.

Fig. 2 shows only schematic examples of sensor signals for an object with a determined mass m, which is lifted and weighed, for example. Assuming the force F(s,t) characteristic for the movement, with position s at time t, the mass may be determined. Signal examples may be supplemented by experiments with several test persons and objects. The obtained signal examples may be used to train a neural network KNN, which may determine the mass m for each event (single up and down movements) with an appropriate confidence interval. Similar examples of sensor signals may be created for an object with a determined mass m that is carried while walking and provided to a neural network KNN for training.

According to a further aspect of the invention, the achievement provides for a corresponding use of a corresponding technical device 100 to determine at least one mass m that is lifted and/or carried by a user B of the technical device 100. According to a further aspect of the invention, the achievement provides a corresponding method for determining at least one mass m that may be lifted and/or carried by a user B, using a corresponding smart device 100. According to a further aspect of the invention, the achievement provides a corresponding computer program product for performing a corresponding method, a corresponding storage medium on which a corresponding computer program product is stored, and a corresponding data carrier signal that transmits a corresponding computer program product. According to a further aspect of the invention, the achievement provides for a corresponding computing unit 30 for performing a corresponding method, wherein, in particular, the computing unit may be used in a corresponding technical device 100.

The foregoing explanation of the embodiments describes the present invention exclusively in the context of examples. Of course, individual features of the embodiments may be freely combined with each other, provided that this is technically reasonable, without leaving the scope of the present invention.

### List of reference signs

- 10: sensor

- 20: storage unit
- 21: storage area
- 22: storage area

- 30: computing unit

- 40: interface

- 100: smart device

- f: frequency examples
- m: mass

- B: user
- K: map

- KNN: artificial neural network

## Claims

1. A method for determining at least one mass (m) that is lifted and/or carried by a user (B), with the aid of a technical device (100), preferably in the form of a smart watch and/or a sports wristband,
wherein the technical device (100) has at least one sensor (10) that is configured to measure at least one dynamic motion parameter,
wherein sensor signals of the at least one sensor (10) are processed and/or analyzed to determine at least one mass (m) lifted and/or carried by the user (B).

2. The method according to the preceding claim,
wherein sensor signals, in particular calibrated and/or normalized sensor signals, of the at least one sensor (10) are processed using a method for frequency analysis in order to determine at least one mass (m),
and/or wherein the frequency, the amplitude and/or the shape of, in particular calibrated and/or normalized, sensor signals of the at least one sensor (10), are analyzed for determining the at least one mass (m),
in order to determine the mass (m) in particular as a function of the frequency, the amplitude and/or the shape, in particular of the transients and/or slopes, of, in particular calibrated and/or normalized, sensor signals of the at least one sensor (10),
and/or wherein at least one filter, for example a high pass filter and/or a low pass filter, is used to determine whether the user (B) is carrying and/or lifting a mass (m).

3. The method according to any one of the preceding claims,
wherein different signal examples and/or frequency examples (f) of, in particular calibrated and/or normalized, sensor signals of the at least one sensor (10), are processed with the aid of a characteristic field (K) in order to determine the mass (m),
and/or wherein sensor signals, in particular calibrated and/or normalized sensor signals, of the at least one sensor (10), are processed with the aid of an artificial neural network (KNN) to determine the mass (m).

4. The method according to any one of the preceding claims,
wherein sensor signals of the at least one sensor (10) are calibrated and/or normalized in dependence on at least one vital parameter of the user (B), in particular comprising: a weight, a body height, a body density, a BMI index, a pulse, a heartbeat, a blood volume, a blood pressure, an oxygen saturation in the blood and/or a temperature,
and/or wherein sensor signals of the at least one sensor (10), are processed and/or analyzed in dependence on at least one geographical position of the user (B),
and/or wherein at least one sensor of the technical device (100) is actuated to measure and/or determine at least one vital parameter and/or at least one geographical position of the user (B).

5. The method according to any one of the preceding claims,
wherein at least one training run is performed
to obtain at least one calibration signal of the at least one sensor (10), for a motion of the user (B) without a mass (m),
which may be used preferably
to calibrate and/or normalize sensor signals of the at least one sensor (10), for a motion of the user (B) with a mass (m),
and/or wherein at least one or more measurements are performed to obtain different signal examples and/or frequency examples (f) of, in particular calibrated and/or normalized, sensor signals of the at least one sensor (10), for corresponding masses (m),
preferably for at least one user (B) or for several different users (B).

6. The method according to any one of the preceding claims,
wherein load profiles are created and/or analyzed,
preferably for at least one user (B) or for several different users (B),
wherein in particular load profiles are created regularly, e.g. when a mass (m) is lifted and/or carried, periodically, e.g. every day, and/or event-controlled, e.g. by activation, e.g. by the user (B).

7. The method according to any one of the preceding claims,
wherein motion profiles are created and/or analyzed for measurement of a liquid intake, preferably for at least one user (B) or for several different users (B),
wherein in particular motion profiles are created regularly, e.g. when a mass (m) is lifted and/or carried, periodically, e.g. every day, and/or event-controlled, e.g. by activation, e.g. by the user (B).

8. The method according to any one of the preceding claims,
wherein a user input, especially a voice command and/or a touch command, are captured in order to start determining at least one mass (m) lifted and/or carried by the user (B) and/or to start creating and/or analyzing at least one load profile of the user (B) and/or to start a measurement of a liquid intake,
and/or wherein at least one specific mass (m) lifted and/or carried by the user (B) and/or at least one load profile of the user (B) and/or a measured liquid intake are output to the user (B).

9. The method according to any one of the preceding claims,
whereby an acoustic, optical and/or haptic feedback is output to the user (B),
if at least one determined mass (m) and/or if at least one created load profile exceeds a certain risk threshold for the user (B),
wherein in particular the feedback may comprise at least one or more warning levels, and/or wherein at least one determined mass (m) lifted and/or carried by the user (B) and/or at least one load profile of the user (B) are/is transmitted to a mobile device of the user (B) and/or to a health service, for example at an employer and/or a physician, and/or an emergency call is sent out if at least one determined mass (m) and/or if at least one created load profile exceeds a certain risk threshold for the user (B).

10. A computer program product comprising instructions which, when the computer program is executed by a computer, cause the computer program to perform a method according to any one of the preceding claims.

11. A storage medium on which the computer program product according to the above claim is stored.

12. A computing unit (30) for determining at least one mass (m) that is lifted and/or carried by a user (B),
comprising a memory device and a computing device,
wherein a code is stored in the memory device,
and wherein upon execution of the code by the computing device, a method according to any one of the preceding claims is performed.

13. A technical device (100), especially a smart device , preferably in the form of a smart watch and/or a wristband, for a user (B) for determining at least one mass (m) that is lifted and/or carried by the user (B),
comprising:
- at least one sensor (10), especially one motion sensor, that is configured to measure at least one dynamic motion parameter, and
- a computing unit (30),
wherein the computing unit (30) is specifically arranged to process and/or analyze sensor signals of the at least one sensor (10) and to determine the at least one mass (m) lifted and/or carried by the user (B) in dependence on the processing and/or the analysis.

14. Use of a technical device (100), preferably a smart device, especially according to any one of the preceding claims, to determine at least one mass (m) lifted and/or carried by a user (B) of the device (100).

15. Use of a technical device (100), preferably a smart device, especially according to any one of the preceding claims, for measurement of a liquid intake.
